(19) Europäisches Patentamt
European Patent Office
Office européen des brevets

(11) EP 1 905 796 A1

(12) EUROPEAN PATENT APPLICATION

(43) Date of publication:
**02.04.2008 Bulletin 2008/14**

(21) Application number: **07018939.4**

(22) Date of filing: **26.09.2007**

(51) Int Cl.:
***C08J 7/04** (2006.01)* ***G01N 33/53** (2006.01)*
***A61J 1/00** (2006.01)* ***A61M 3/00** (2006.01)*

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: **29.09.2006 JP 2006267938**

(71) Applicant: **FUJIFILM Corporation**
**Minato-ku**
**Tokyo (JP)**

(72) Inventors:
- **Kubo, Takashi**
**Kaisei-machi**
**Ashigarakami-gun**
**Kangawa, 258-8577 (JP)**
- **Nishimi, Taisei**
**Kaisei-machi**
**Ashigarakami-gun**
**Kangawa, 258-8577 (JP)**
- **Hoshi, Satoshi**
**Kaisei-machi**
**Ashigarakami-gun**
**Kangawa, 258-8577 (JP)**

(74) Representative: **HOFFMANN EITLE**
**Patent- und Rechtsanwälte**
**Arabellastrasse 4**
**81925 München (DE)**

# (54) Biochemical instrument

(57) It is an object of the present invention to provide a biochemical instrument wherein an adhesive layer that has hydrophilic properties, heat resistance, and satisfactory adhesiveness is provided between a plastic material surface and a hydrophilic polymer layer. The present invention provides a biochemical instrument comprising a plastic material that is coated on its surface with a hydrophilic polymer, which comprises a metal oxide thin film between the plastic material and the hydrophilic polymer layer, wherein the film stress of said metal oxide thin film is less than $5.4 \times 10^{21}$ dyn/cm$^2$.



Printed by Jouve, 75001 PARIS (FR)

## Description

Technical Field

The present invention relates to a biochemical instrument comprising a plastic material coated with a hydrophilic polymer.

Background Art

Various types of biochemical instruments (e.g., containers, flow channels, pipettes, and syringes) are often brought into surface contact with solutions containing biologically-relevant substances (e.g., blood, cells, and proteins) or hydrophobic low-molecular-weight compounds (e.g., pharmaceutical compounds and surfactants). It is preferable that such surfaces do not allow nonspecific adsorption of biologically-relevant substances or hydrophobic low-molecular-weight compounds. In order to inhibit nonspecific adsorption of biologically-relevant substances or hydrophobic low-molecular-weight compounds, hydrophobic surfaces of plastic materials may be converted into hydrophilic surfaces by coating such surfaces with a hydrophilic polymer. When hydrophobic surfaces of plastic materials are coated with a hydrophilic polymer, adhesive layers may be provided between hydrophobic surfaces and hydrophilic polymer coatings. Such adhesive layers are required to have heat resistance and good adhesion properties as well as hydrophilic properties. However, adhesive layers that satisfy such requirements have not yet been examined. At present, surfaces that can inhibit nonspecific adsorption of both the biologically-relevant substances and hydrophobic low-molecular-weight compounds have not yet been developed.

JP Patent Publication (kokai) No. 5-132574 A (1993) discloses a method for producing anti-fog plastics which comprises applying a solution containing a silane compound to a plastic substrate surface coated with a metal oxide layer having $SiO_x$ (where x is 1 or 2), treating the resulting silane coupling layer with a modifying solution containing a primary amine derivative having functional groups to form a hydrophilic organic layer on the surface, and binding the functional groups of a primary amine derivative with a crosslinking agent. JP Patent Publication (kokai) No. 5-132574 A (1993), however, does not involve the use of a hydrophilic polymer.

JP Patent Publication (kokai) No. 2000-137195 A discloses a device which comprises a biomedical device comprising, on at least one of its surfaces, one or more types of functional silane coupling agents in amounts effective for a reaction and one or more types of hydrophilic polymers in amounts effective for coating. However, JP Patent Publication (kokai) No. 2000-137195 A does not disclose a metal oxide.

Disclosure of the Invention

The present invention is directed to overcoming the aforementioned drawbacks of conventional techniques. Specifically, it is an object of the present invention to provide a biochemical instrument wherein an adhesive layer that has hydrophilic properties, heat resistance, and satisfactory adhesiveness is provided between a plastic material surface and a hydrophilic polymer layer. It is another object of the present invention to provide a biochemical instrument having surfaces that can inhibit both adsorption of biopolymers and adsorption of hydrophobic low-molecular-weight compounds.

The present inventors have conducted concentrated studies in order to attain the above objects. As a result, they discovered that a biochemical instrument having desired properties could be realized by providing a biochemical instrument comprising a plastic material that is coated, on its surface, with a hydrophilic polymer and a metal-oxide thin film between the plastic material and the hydrophilic polymer layer and adjusting the film stress of the metal-oxide thin film to less than $5.4 \times 10^{21}$ (dyn/cm$^2$). This has led to the completion of the present invention.

Specifically, the present invention provides a biochemical instrument comprising a plastic material that is coated on its surface with a hydrophilic polymer, which comprises a metal oxide thin film between the plastic material and the hydrophilic polymer layer, wherein the film stress of said metal oxide thin film is less than $5.4 \times 10^{21}$ dyn/cm$^2$.

Preferably, the plastic material has a thermal expansion coefficient ($K^{-1}$) of 1.3 to 15 $\times$ $10^{-5}$,

Preferably, the plastic material is made from polymethylmethacrylate (PMMA), polycarbonate (PC), cycloolefin polymer (COP), polyethylene naphthalate (PEN), polyethylene terephthalate (PET), polypropylene (PP), polyimide (PI), liquid crystal polyester (LCP), or polydimethylsiloxane (PDMS).

Preferably, the metal oxide is $SiO_2$, $Al_2O_3$, $TiO_2$, $MnO_2$, MgO, $SnO_2$, NiO, $GeO_2$, or a mixture thereof.

Preferably, the thickness of the metal oxide film is between 10 nm and 500 nm.

Preferably, the hydrophilic polymer layer has a portion that inhibits adsorption of hydrophobic low-molecular-weight compounds and a portion that inhibits adsorption of biopolymers.

Preferably, the portion that inhibits adsorption of hydrophobic low-molecular-weight compounds is composed of an inorganic crosslinking polymer, an organic crosslinking polymer, or an organie/inorganic hybrid polymer.

Preferably, the portion that inhibits adsorption of hydrophobic low-molecular-weight compounds comprises a

hydrolytic condensate of metal alkoxide.

Preferably, the portion that inhibits adsorption of biopolymers is composed of a hydrophilic polymer.

Preferably, the hydrophilic polymer is a non-charged hydrophilic polymer.

Preferably, the non-charged hydrophilic polymer is any of polyethylene glycol, polyvinylpyrrolidone, polyvinyl alcohol, poly(hydroxylalkyl)methacrylate, polyacrylamide, MPC polymer, dextran, agarose, pullulan, or polypeptide.

Preferably, the portion that inhibits adsorption of hydrophobic low-molecular-weight compounds and the portion that inhibits adsorption of biopolymers each have a structure represented by the following formula:

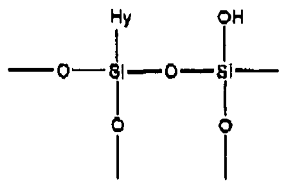

wherein Hy represents a hydrophilic polymer,

In the above formula, preferably, Hy is any of polyethylene glycol, polyvinylpyrrolidone, polyvinyl alcohol, poly (hydroxylalkyl)methacrylate, polyacrylamide, MPC polymer, dextran, agarose, pullulan, or polypeptide,

Preferably, the biochemical instrument of the present invention is obtained by coating the surface of the plastic material with a solution containing a polymer represented by formula (I) or (II) and allowing the polymer to crosslink.

Formula (I)

$$(R^1)_x(OR^2)_{3-x}Si-L_1-(A)-L_2-Si(OR^3)_{3-y}(R^4)_y$$

Formula (II)

$$(R^5)_z(OR^6)_{3-z}Si-L_3-(B)-R^7$$

wherein $R^1$ to $R^6$ each independently represent a hydrogen atom or a hydrocarbon group containing 8 or fewer carbon atoms; $R^7$ represents a hydrogen atom or a monovalent nonmetal atomic group; x, y, and z each independently are an integer from 0 to 2; $L^1$ to $L^3$ each independently represent a divalent linking group containing 3 or more types of atoms selected from among carbon, hydrogen, oxygen, nitrogen, and sulfur atoms; and A and B each independently represent a polymer or an oligomer having structural unit repeats.

The other aspect of the present invention provides a biosensor which comprises the above-described biochemical instrument of the present invention.

Brief Description of the Drawings

Fig. 1 shows the determination of a radius of curvature R by measurement of substrate warpage using an interference microscope.

Fig, 2 shows the film stress and the contact angle resulting from the use of various materials and vapor-deposition techniques.

Best Modes for Carrying out the Invention

Hereafter, the present invention is described in greater detail.

The biochemical instrument of the present invention comprises a plastic material that is coated on its surface

with a hydrophilic polymer, and is characterized in that a metal oxide thin film is present between the plastic material and the hydrophilic polymer layer, and the film stress of the metal oxide thin film is less than 5.4 x $10^{21}$ dyn/$cm^2$.

Examples of metal oxide that can be used include $SiO_2$, SiO, $Al_2O_3$, $TiO_2$, $MnO_2$, MgO, $ArO_2$, CaO, $SnO_2$, NiO, $GeO_2$, $In_2$ $O_3$, $WO_3$, $MoO_3$, $Ta_2O_5$, $HfO_2$, and BaO. They may be used alone or in combinations of two or more. Preferably, $SiO_2$, $Al_2O_3$, $TiO_2$, $MnO_2$, MgO, $SnO_2$, NiO, $GeO_2$, or a mixture thereof can be used as metal oxide. In the present invention, the metal oxide thin film may be a monolayer composed of any one of the aforementioned materials, a monolayer composed of two or more of the aforementioned materials, or a multilayer composed of two or more such monolayers. In the present invention, it is particularly preferable that a metal oxide thin film of $SiO_2$ or SiO or a metal oxide thin film prepared from a composition containing $SiO_2$ or SiO be provided.

The thickness of the metal oxide thin film can be adequately determined, When the plastic material surface is coated with a $SiO_2$ or SiO thin film via vacuum vapor-deposition, for example, the thickness of the metal oxide thin film is preferably between 10 nm and 500 nm. If the layer is too thin, the surface of the plastic material cannot be completely and uniformly coated. On the contrary, cracking or delamination occurs between metal oxide layers if the layer is too thick. Thus, the layer thickness outside the aforementioned range is not desirable.

In the present invention, the film stress of the metal oxide thin film is less than 5.4 x $10^{21}$ dyn/$cm^2$ and preferably not more than 3.8 x $10^{21}$ yn/$cm^2$. The film stress ($\sigma$) is represented by the equation: $\sigma$ = K/(rxd) wherein K represents a constant; r represents the radius of curvature of a substrate to which the layer adheres; and d represents film thickness.

The method for preparing a metal oxide thin film is not particularly limited. For example, physical deposition such as vacuum deposition or sputtering, the CVD method, or plating may be employed, with vacuum deposition being preferable.

Specific examples of the biochemical instrument (e.g., an instrument for biochemical assay) of the present invention include a beaker, a flask, a petri dish, a pipette, a syringe, a centrifuge tube, a needle, a tube, a Eppendorf chip, a titer plate, a micro-flow channel, and a filter. It should be noted that the biochemical instrument of the present invention is not limited thereto and includes any instrument that may be brought into contact with a solution containing biologically-relevant substances (e.g., blood, cells, and proteins) or hydrophobic low-molecular-weight compounds (e.g., pharmaceutical compounds and surfactants).

The plastic material that constitutes the biochemical instrument of the present invention is not particularly limited. For example, a common material that is used for a biochemical instrument can be used. The thermal expansion coefficient ($K^{-1}$) of the plastic material that is used in the present invention is preferably 1.3 to 15 $\times$ $10^{-5}$. The thermal expansion coefficient refers to the degree of expansion of a substance resulting from a temperature increase of 1K (°C).

Specific examples of a plastic material include, but are not limited to, polymethylmethacrylate (PMMA), polycarbonate (PC), cycloolefin polymer (COP), polyethylene naphthalate (PEN), polyethylene terephthalate (PET), polypropylene (PP), polyimide (PI), liquid crystal polyester (LCP), and polydimethylsiloxane (PDMS).

According to a preferable embodiment of the present invention, the hydrophilic polymer layer comprises the portion that inhibits adsorption of hydrophobic low-molecular-weight compounds and the portion that inhibits adsorption of biopolymers. In such a case, the portion that inhibits adsorption of hydrophobic low-molecular-weight compounds and the portion that inhibits adsorption of biopolymers are present on the surface of the plastic material. According to a preferable embodiment of the present invention, the portion that inhibits adsorption of biopolymers is located at a site farther from the surface of the water-insoluble material than the portion that inhibits adsorption of hydrophobic low-molecular-weight compounds and/or at a site in the aforementioned portion that inhibits adsorption of hydrophobic low-molecular-weight compounds. That is, in the present invention, the portion that inhibits adsorption of hydrophobic low-molecular-weight compounds may be laminated with the portion that inhibits adsorption of biopolymers, or the portion that inhibits adsorption of biopolymers may be in the portion that inhibits adsorption of hydrophobic low-molecular-weight compounds so as to form a hybrid structure.

Examples of substances, absorption of which is to be inhibited in the present invention, include substances described in "Saibou-seibutsugaku-jiten (Encyclopedia of cell biology," Asakura Publishing Co., Ltd, pp. 148 to 149. The term "hydrophobic low-molecular-weight compound" preferably refers to a compound having a molecular weight of 2,000 or smaller. Specific examples thereof include a drug (e.g., a medicine, narcotic drug, or explosive), a surfactant, and a lipid. Also, preferable examples of biopolymers that are targets of inhibiting adsorption in the present invention include polymers having a molecular weight of 5,000 or greater. Specific examples thereof include a protein, an antibody, a nucleic acid (DNA, RNA), and a polysaccharide.

The term "inhibition (or inhibit)" used herein refers to reduction of the degree of adsorption of biopolymers or hydrophobic low-molecular-weight compounds adsorbed to water-insoluble materials that have not been subjected to a variety of processing procedures.

In this description, "the portion that inhibits adsorption of hydrophobic low-molecular-weight compounds" may have any configuration, area, or volume, provided that such portion is capable of inhibiting adsorption of hydrophobic low-molecular-weight compounds. Also, "the portion that inhibits adsorption of biopolymers" may have any configuration, area, or volume, provided that such portion is capable of inhibiting adsorption of biopolymers, The coverage of the

surface of a water-insoluble material by the portion that inhibits adsorption of hydrophobic low-molecular-weight compounds and the portion that inhibits adsorption of biopolymers is preferably at least 90%, more preferably at least 95%, and particularly preferably 100%.

In the present invention, specific examples of the portion that inhibits adsorption of hydrophobic low-molecular-weight compounds include an inorganic crosslinking polymer and an organic crosslinking polymer. For example, a hydrolytic condensate of metal alkoxide may be used.

In the present invention, an inorganic crosslinking polymer preferably has a three-dimensionally crosslinked structure containing a metal-oxygen bond, such as Ti-O, Si-O, Zr-O, Mn-O, Ce-O, Ba-O, or Al-O. Such inorganic crosslinking polymer is formed by a technique that is referred to as the sol-gel technique, i.e., a technique wherein a metal alkoxide compound is allowed to react with water to convert an alkoxy group in the metal alkoxide compound into a hydroxyl group, and a polymer containing a hydroxy-metal group resulting from polycondensation is subjected to dehydration or dealcoholization to form a three-dimensionally crosslinked structure via a covalent bond. Examples of metal alkoxides that can be used in the sol-gel technique include compounds comprising lower alkoxy groups such as methoxy, ethoxy, isopropoxy, propoxy, isobutoxy, butoxy, or tert-butoxy groups. A metal alkoxide that forms an inorganic crosslinking polymer may be an alkyl-substituted compound in which some alkoxy groups may comprise functional groups such as a silane coupling agent. In the sol-gel technique, various solvents, such as polar solvents such as an alcohol (e.g., ethyl alcohol, isopropyl alcohol, or butyl alcohol) or ketone, or various organic solvents such as hydrocarbon or halogenated hydrocarbon solvents; can be used. In order to accelerate the reaction, the metal alkoxide in the solution may be subjected to partial hydrolysis in advance. In order to accelerate hydrolysis, a small amount of water and/or acid of a hydrolytic catalyst may be added to the solution of metal alkoxide.

In the present invention, an organic crosslinking polymer is provided so as to inhibit adsorption of hydrophobic low-molecular weight compounds. Accordingly, such organic crosslinking polymer is preferably prepared via three-dimensional crosslinking of hydrophilic polymers. Conventional techniques can be employed in order to perform the three-dimensional crosslinking of hydrophilic polymers. Examples of techniques that can be preferably employed include, but are not limited to: a method described in paragraphs 0033 to 0045 of JP Patent Publication (kokai) No. 2004-314073 (A), wherein a polysaccharide is three-dimensionally crosslinked using an acid-catalyst-based N-alkyloxy crosslinking agent; a method described in paragraphs 0009 to 0015 of JP Patent Publication (kokai) No. 2000-301837 A, wherein a polyvinyl alcohol is three-dimensionally crosslinked using a crosslinking agent; a method described in paragraphs 0011 to 0012 of JP Patent Publication (kokai) No. 5-230101 A (1993), wherein a polysaccharide is three-dimensionally crosslinked using a crosslinking agent; a method described in paragraphs 0012 to 0043 of JP Patent Publication (kokai) No. 8-120003 A (1996), wherein a hydrophilic monomer is three-dimensionally crosslinked with a crosslinking unsaturated monomer via copolymerization; a method described in paragraphs 0013 to 0020 of JP Patent Publication (kokai) No. 2004-58566 A, wherein N-vinyl carboxylic acid amide is three-dimensionally crosslinked with a crosslinking agent via electron application; and a method described in paragraphs 0006 to 0016 of JP Patent Publication (kokai) No. 9.87395 A (1997), wherein a polyvinyl alcohol is three-dimensionally crosslinked via electron application.

The portion that inhibits adsorption of biopolymers is preferably composed of a hydrophilic polymer and more preferably a non-charged hydrophilic polymer. The term "non-charged" used herein refers to an electric charge that is not strong enough to electrostatically suction and/or electrostatically repulse biopolymers or hydrophobic low-molecular-weight compounds. Such portion may have a weak electric charge that would not provide electrostatic interaction. Specific examples of a non-charged hydrophilic polymer include, but are not limited to, polyethylene glycol, polyvinylpyrrolidone, polyvinyl alcohol, poly(hydroxyalkyl)methacrylate, polyacrylamide, a polymer comprising a phosphoryl choline group in a side chain, a polysaccharide, and polypeptide.

In the present invention, specific examples of the portion that inhibits adsorption of hydrophobic low-molecular-weight compounds and the portion that inhibits adsorption of biopolymers include those obtained by crosslinking the compounds represented by formulae (I) and (II),

Formula (I)

$$(R^1)_x(OR^2)_{3-x}Si-L_1-(A)-L_2-Si(OR^3)_{3-y}(R^4)_y$$

Formula (II)

$$(R^5)_z(OR^6)_{3-z}Si-L_3-(B)-R^7$$

Also, a substance that is obtained by crosslinking the compounds represented by formulae (III) and (IV) preferably can be used.

Formula (III)

$$(R^1)_x(OR^2)_{3-x}Si-L_1-(CH_2CH_2O)_n-L_2-Si(OR^3)_{3-y}(R^4)_y$$

Formula (IV)

$$(R^5)_z(OR^6)_{3-z}Si-L_3-(CH_2CH_2O)_m-R^7$$

$n$=3 to 25,000, $m$=3 to 25,000

In formulae (I), (II), (III), and (IV), $R^1$ to $R^6$ each independently represent a hydrogen atom or a hydrocarbon group containing 8 or fewer carbon atoms, $R^7$ represents a hydrogen atom or a monovalent nonmetal atomic group, and x, y, and z each independently are an integer from 0 to 2. In compounds represented by formulae (I) and (III), portions represented by $-Si(OR^2)_{3-x}(R^1)_x$ and $-Si(OR^3)_{3-y}(R^4)_y$ are polymerized via crosslinking, and portions represented by $-Si(OR^6)_{3-z}(R^5)_z$ in the compounds represented by formulae (II) and (IV) are polymerized via crosslinking (where $OR^2$, $OR^3$, and $OR^5$ are discharged outside the system via hydrolysis and $R^1$, $R^4$, and $R^5$ are crosslinked with Si) to form the portion that inhibits adsorption of hydrophobic low-molecular-weight compounds. $L^1$ to $L^3$ each independently represent a divalent linking group containing 3 or more types of atoms selected from among carbon, hydrogen, oxygen, nitrogen, and sulfur atoms.

In formula (I) and (II), A and B each independently represent a polymer or an oligomer comprising structural unit repeats.

Examples of hydrocarbon groups represented by $R^1$ to $R^6$ include alkyl and aryl groups, with a linear, branched, or cyclic alkyl group having 1 to 8 carbon atoms being preferable. Specific examples thereof include methyl, ethyl, propyl, butyl, pentyl, hexyl, heptyl, octyl, isopropyl, isobutyl, s-butyl, t-butyl, isopentyl, neopentyl, 1-methylbutyl, isohexyl, 2-ethylhexyl, 2-methylhexyl, and cyclopentyl groups.

$R^1$ to $R^6$ preferably each represent a hydrogen atom, a methyl group, or an ethyl group from the viewpoints of effects and availability.

Such hydrocarbon group may be optionally substituted. When an alkyl group is substituted, a substituted alkyl group is formed by a bond between a substituent and an alkylene group. A substituent is a monovalent nonmetal atomic group excluding hydrogen. Preferable examples thereof include a halogen atom (-F, -Br, -C1, -I), a hydroxyl group, an alkoxy group, an aryloxy group, a mercapto group, an alkylthio group, an arylthio group, an alkyldithio group, an aryldithio group, an amino group, an N-alkylamino group, an N,N-diarylamino group, an N-alkyl-N-arylamino group, an acyloxy group, a carbamoyloxy group, an N-alkylcarbamoyloxy group, an N-arylcarbamoyloxy group, an N,N-dialkylcarbamoyloxy group, an N,N-diarylcarbamoyloxy group, an N-alkyl-N-arylcarbamoyloxy group, an alkylsulfoxy group, an arylsulfoxy group, an acylthio group, an acylamino group, an N-alkylacylamino group, an N-arylacylamino group, an ureide group, an N'-alkylureide group, an N',N'-dialkylureide group, an N'-arylureide group, an N',N'-diarylureide group, an N'-alkyl-N'-arylureide group, an N-alkylureide group, an N-arylureide group, an N'-alkyl-N-alkylureide group, an N'-alkyl-N-arylureide group, an N',N'dialkyl-N-alkylureide group, an N',N'-dialkyl-N-arylureide group, an N'-aryl-N-alkylureide group, an N'-aryl-N-arylureide group, an N',N'-diaryl-N-alkylureide group, an N',N'-diaryl-N-arylureide group, an N'-alkyl-N'-aryl-N-alkylureide group, an N'-alkyl-N'-aryl-N-arylureide group, an alkoxycarbonylamino group, an aryloxycarbonylamino group, an N-alkyl-N-alkoxycarbonylamino group, an N-alkyl-N-aryloxycarbonylamino group, an N-aryl-N-alkoxycarbonylamino group, an N-aryl-N-aryloxycarbonylamino group, an formyl group, an acyl group, an carboxyl group, an alkoxycarbonyl group, an aryloxycarbonyl group, an carbamoyl group, an N-alkylcarbamoyl group, an N,N-dialkylcarbamoyl group, an N-arylcarbamoyl group, an N,N-diarylcarbamoyl group, an N-alkyl-N-arylcarbamoyl group, an alkylsulfinyl group, an arylsulfinyl group, an alkylsulfonyl group, an arylsulfonyl group, an sulfo group ($-SO_3H$) and a conjugate base group thereof (hereafter abbreviated as a "sulfonate group"), alkoxysulfonyl group, an aryloxysulfonyl group, an sulfinamoyl group, an N-alkylsulfinamoyl group, an N,N-dialkylsulfinamoyl group, an N-arylsulfinamoyl group, an N,N-diarylsulfinamoyl group, an N-alkyl-N-arylsulfinamoyl group, an sulfamoyl group, an N-alkylsulfamoyl group, an N,N-dialkylsulfamoyl group, an N-arylsulfamoyl group, an N,N-diarylsulfamoyl group, an N-alkyl-N-arylsulfamayl group, an phosphono group ($-PO_3H_2$) and a conjugate base group thereof (hereafter abbreviated as a "phosphonate group"), dialkylphosphono group ($-PO_3(alkyl)_2$), diarylphosphono group ($-PO_3(aryl)_2$), alkylarylphosphono group ($-PO_3(alkyl)(aryl)$), monoalkylphosphono group ($-PO_3H(alkyl)$) and a conjugate base group thereof (hereafter abbreviated as a "alkylphosphonate group"), monoarylphosphono group ($-PO_3H(aryl)$) and a conjugate base group thereof (hereafter abbreviated as a "arylphosphonate group"), phosphonooxy group ($-OPO_3H_2$) and a conjugate base group thereof (hereafter abbreviated as a "phosphonateoxy group"), dialkylphosphonooxy group ($-OPO_3(alkyl)_2$), diarylphosphonooxy group ($-OPO_3(aryl)_2$), alkylarylphosphonooxy group (-OPO(alkyl)(aryl)), monoalkylphosphonooxy group ($-OPO_3H(alkyl)$) and a conjugate base group thereof (hereafter abbreviated as a "alkylphosphonateoxy group"), monoarylphosphonooxy group ($-OPO_3H(aryl)$) and a conjugate base group thereof (hereafter abbreviated as a "arylphosphonateoxy group"), morpholino group, an cyano group, an nitro group, an aryl group, an alkenyl group, and an alkynyl groups.

Specific examples of alkyl in these substituents include examples of alkyl groups defined concerning $R^1$ to $R^6$. Specific examples of aryl groups include a phenyl group, a biphenyl group, a naphthyl group, a ditolyl group, a xylyl group, a mesityl group, a cumenyl group, a chlorophenyl group, a bromophenyl group, a chloromethylphenyl group, a hydroxyphenyl group, a methoxyphenyl group, an ethoxyphenyl group, a phenoxyphenyl group, an acetoxyphenyl group, a benzoyloxyphenyl group, a methylthiophenyl group, a phenylthiophenyl group, a methylaminophenyl group, a dimethylaminophenyl group, an acetylaminophenyl group, a carboxyphenyl group, a methoxycarbonylphenyl group, a ethoxyphenylcarbonyl group, a phenoxycarbonylphenyl group, an N-phenylcarbamoylphenyl group, a phenyl group, a cyanophenyl group, a sulfophenyl group, a sulfonatephenyl group, a phosphonophenyl group, and a phosphonatephenyl group. Examples of alkenyl groups include a vinyl group, 1-propenyl group, a 1-butenyl group, a cinnamyl group, and 2-chloro-1-ethenyl group. Examples of alkynyl groups include an ethynyl group, 1-propinyl group, 1-butynyl group, and a trimethylsilylethynyl group. Examples of $G^1$ in the acyl group ($G^1CO-$) include hydrogen and the aforementioned aryl and alkyl groups.

Among these substituents, examples of more preferable substituents include a halogen atom (-F, -Br, -Cl, -I), an alkoxy group, an aryloxy group, an alkylthio group, an arylthio group, an N-alkylamino group, an N,N-dialkylamino group, an acyloxy group, an N-alkylcarbamoyloxy group, an N-arylcarbamoyloxy group, an acylamino group, an formyl group, an acyl group, an carboxyl group, an alkoxycarbonyl group, an aryloxycarbonyl group, an carbamoyl group, an N-alkylcarbamoyl group, an N,N-dialkylcarbamoyl group, an N-arylcarbamoyl group, an N-alkyl-N-arylcarbamoyl group, a sulfo group, a sulfonate group, a sulfamoyl group, an N-alkylsulfamoyl group, an N,N-dialkylsulfamoyl group, an N-arylsulfamoyl group, an N-alkyl-N-arylsulfamoyl group, a phosphono group, a phosphonate group, a dialkylphosphono group, a diarylphosphono group, a monoalkylphosphono group, an alkylphosphonate group, an monoarylphosphono group, an arylphosphonate group, an phosphonooxy group, an phosphonateoxy group, an aryl group, and an alkenyl group.

An example of an alkylene group in the substituted alkyl group is a group which is obtained by removing any one of the hydrogen atoms of the alkyl group having 1 to 20 carbon atoms so as to prepare a divalent organic residue. A preferable example is a linear alkylene group comprising 1 to 12 carbon atoms, a branched alkylene group comprising 3 to 12 carbon atoms, and a cyclic alkylene group having 5 to 10 carbon atoms. Specific examples of preferable substituted alkyl group which is obtained by combining such substituent and an alkylene group include a chloromethyl group, a bromomethyl group, a 2-chloroethyl group, a trifluoromethyl group, a methoxymethyl group, a methoxyethoxyethyl group, an allyloxymethyl group, a phenoxymethyl group, a methylthiomethyl group, a tolylthiomethyl group, an ethylaminoethyl group, a diethylaminopropyl group, a morpholinopropyl group, an acetyloxymethyl group, a benzoyloxymethyl group, an N-cyclohexylcarbamoyloxyethyl group, an N-phenylcarbamoyloxyethyl group, an acetylaminoethyl group, an N-methylbenzoylaminopropyl group, a 2-oxyethyl group, a 2-oxypropyl group, a carboxypropyl group, a methoxycarbonylethyl group, an allyloxycarbonylbutyl group, a chlorophenoxycarbonylmethyl group, a carbamoylmethyl group, an N-methylearbamoylethyl group, an N,N-dipropylcarbamoylmethyl group, an N-(methoxyphenyl)carbamoylethyl group, an N-methyl-N-(sulfophenyl)carbamoylmethyl group, a sulfobutyl group, a sulfonatebutyl group, a sulfamoylbutyl group, an N-ethylsulfamoylmethyl group, an N,N-dipropylsulfamoylpropyl group, an N-tolylsulfamoylpropyl group, an N-methyl-N-(phosphonophenyl)sulfarnoyloctyl group, a phosphonobutyl group, a phosphonatehexyl group, a diethylphosphonobutyl group, a diphenylphosphonopropyl group, a methylphosphonobutyl group, a methylphosphonatebutyl group, a tolylphosphonohexyl group, a tolylphosphonatehexyl group, a phosphonooxypropyl group, a phosphonateoxybutyl group, a benzyl group, a phenetyl group, an α-methylbenzyl group, a 1-mothyl-1-phenylethyl group, a p-methylbenzyl group,

a cinnamyl group, an allyl group, a 1-propenylmethyl group, a 2-butenyl group, a 2-methylallyl group, a 2-methylpropenylmethyl group, a 2-propynyl group, a 2-butynyl group, and a 3-butynyl group.

x, y, and z each independently are an integer of 0, 1, or 2, with 0 being particularly preferable.

$R^7$ represents the portion that inhibits adsorption of biopolymers when the compounds represented by formulae (I) to (IV) are crosslinked. Examples of monovalent nonmetal atomic groups represented by $R^7$ include monovalent nonmetal atomic groups listed as specific examples of $R^1$ to $R^6$. Specific examples of preferable groups include a methyl group, an ethyl group, a propyl group, a butyl group, a pentyl group, a hexyl group, a heptyl group, an octyl group, an isopropyl group, an isobutyl group, an s-butyl group, a t-butyl group, an isopentyl group, a neopentyl group, a 1-methylbutyl group, an isohexyl group, a 2-ethylhexyl group, a 2-methylhexyl group, a cyclopentyl group, a halogen atom, and a hydroxyl group. From the viewpoint of improved hydrophilic properties and availability, a hydrogen atom, a methyl group, and a hydroxyl group are preferable,

$L^1$ to $L^3$ each independently represent a divalent linking group comprising 3 or more types of atoms selected from among a carbon atom, a hydrogen atom, an oxygen atom, a nitrogen atom, and a sulfur atom. Specifically, $L^1$ to $L^3$ are each composed of 1 to 60 carbon atoms, 0 to 10 nitrogen atoms, 0 to 50 oxygen atoms, 1 to 100 hydrogen atoms, and 0 to 20 sulfur atoms, Further specific examples of linking groups include the following structural units or those composed of two or more of such units in combination,

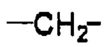 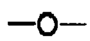 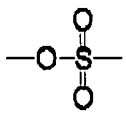 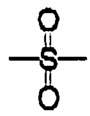 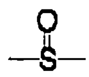

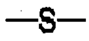 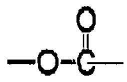 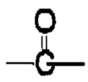 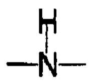

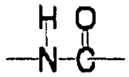 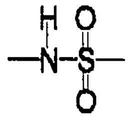 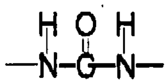 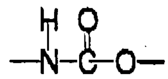

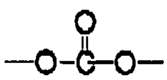 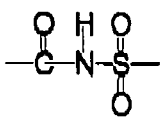

A and B each independently represent a hydrophilic polymer or a hydrophilic oligomer having structural unit repeats, and is the portion that inhibits adsorption of biopolymers. Specifically, A and B each preferably represent polyethylene glycol, polyvinylpyrrolidone, polyvinyl alcohol, poly(hydroxylalkyl)methacrylate, polyacrylamide, a polymer comprising a phosphoryl choline group in the side chain, a polysaccharide, or polypeptide.

Such polymer and oligomer may comprise a single type of a structural unit or two or more types of structural units.

The molecular weight of an organosilicon compound represented by any of formulae (I) to (IV) is preferably 100 to 1,000,000, more preferably 400 to 500,000, and most preferably 1,000 to 200,000. In order to bring the molecular weight to such a preferable range, the structure of the alkoxysilyl group at the terminus, the structure and the molar ratio of polymerization of the polymer and the oligomer represented by A and B, and the number of instances of polymerization may be determined.

In the present invention, particularly preferable examples of compounds represented by formula (III) are polymers represented by the following formulae:

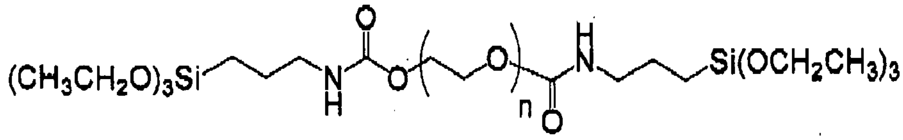

**Polymer 1**

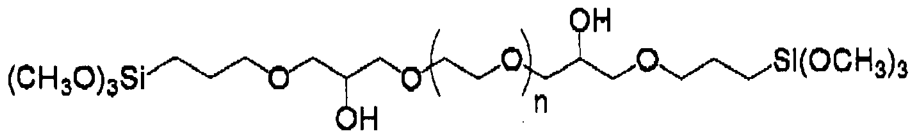

**Polymer 2**

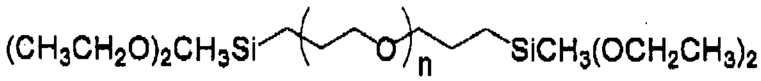

**Polymer 3**

wherein n is an integer between 3 and 25,000.

In the present invention, particularly preferable examples of compounds represented by formula (IV) are polymers represented by the following formulae:

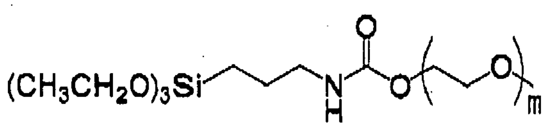

**Polymer 4**

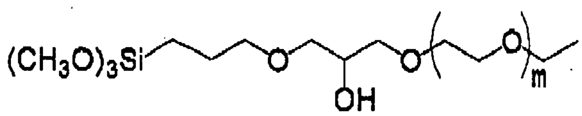

**Polymer 5**

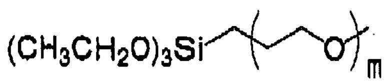

**Polymer 6**

wherein m is an integer between 3 and 25,000.

In the present invention, the most preferable example of a compound represented by formula (IV) is a polymer

represented by the above formula for Polymer 4.

The biochemical instrument of the present invention can be used as a member that constitutes a biosensor (e.g., a detector plane or a flow channel).

The biosensor of the present invention has as broad a meaning as possible, and the term biosensor is used herein to mean a sensor, which converts an interaction between biomolecules into a signal such as an electric signal, so as to measure or detect a target substance. The conventional biosensor is comprised of a receptor site for recognizing a chemical substance as a detection target and a transducer site for converting a physical change or chemical change generated at the site into an electric signal. In a living body, there exist substances having an affinity with each other, such as enzyme/substrate, enzyme/coenzyme, antigen/antibody, or hormone/receptor. The biosensor operates on the principle that a substance having an affinity with another substance, as described above, is immobilized on a substrate to be used as a molecule-recognizing substance, so that the corresponding substance can be selectively measured.

In the biosensor of the present invention, a metal surface or metal film can be used as a substrate. A metal constituting the metal surface or metal film is not particularly limited, as long as surface plasmon resonance is generated when the metal is used for a surface plasmon resonance biosensor. Examples of a preferred metal may include free-electron metals such as gold, silver, copper, aluminum or platinum. Of these, gold is particularly preferable. These metals can be used singly or in combination. Moreover, considering adherability to the above substrate, an interstitial layer consisting of chrome or the like may be provided between the substrate and a metal layer.

The film thickness of a metal film is not limited. When the metal film is used for a surface plasmon resonance biosensor, the thickness is preferably between 0.1 nm and 500 nm, and particularly preferably between 1 nm and 200 nm. If the thickness exceeds 500 nm, the surface plasmon phenomenon of a medium cannot be sufficiently detected. Moreover, when an interstitial layer consisting of chrome or the like is provided, the thickness of the interstitial layer is preferably between 0.1 nm and 10 nm.

Formation of a metal film may be carried out by common methods, and examples of such a method may include sputtering method, evaporation method, ion plating method, electroplating method, and nonelectrolytic plating method.

A metal film is preferably placed on a substrate. The description "placed on a substrate" is used herein to mean a case where a metal film is placed on a substrate such that it directly comes into contact with the substrate, as well as a case where a metal film is placed via another layer without directly coming into contact with the substrate. When a substrate used in the present invention is used for a surface plasmon resonance biosensor, examples of such a substrate may include, generally, optical glasses such as BK7, and synthetic resins. More specifically, materials transparent to laser beams, such as polymethyl methacrylate, polyethylene terephthalate, polycarbonate or a cycloolefin polymer, can be used. For such a substrate, materials that are not anisotropic with regard to polarized light and have excellent workability are preferably used.

The present invention is described in greater detail with reference to the following examples, although the technical scope of the present invention is not limited thereto.

Examples

Test Example 1:

In order to inspect the correlation between the film stress of a vapor-deposited membrane and delamination and cracking of the film, various types of inorganic oxide substances were vacuum vapor-deposited or sputter-deposited on a silicone substrate (thickness: 0,625 mm; length: 75 mm; width: 26 mm) to a film thickness of 100 nm, In connection with film stress, a radius of curvature R was determined by measuring the substrate warpage using an interference microscope as shown in Fig. 1. The radius of curvature R that indicates the substrate warpage is defined by the following equation:

$$R = Es \cdot ts^2/6(1\text{-}vs)\sigma tf$$

wherein Es represents the Young's modulus of the substrate; ts represents a substrate thickness; vs represents the Poisson's ratio of the substrate; σ represents the film stress, and tf represents the thin film thickness, provided that the condition is similar to the formula: ts (thickness of substrate) >> tf (thickness of thin film).

Thus, the film stress σ is represented by the equation: $\sigma = Es \cdot ts^2/6(1\text{-}vs)Rtf$.

The silicone monocrystalline substrate used has a Young's modulus Es of $1.9 \times 10^{12}$ dyn/cm$^2$, a Poisson's ratio vs of 0.2, a substrate thickness ts of $0.625 \times 10^{-3}$m, and a thin film thickness tf of $10^2 \times 10^{-9}$ m. Thus, the film stress is as follows:

$$\sigma = 1.54 \times 10^{23} \times 1/R \ (dyn/cm^2).$$

The film stress and delamination of the vapor-deposited film were measured in the following manner.

1. Film stress

A thin film (100 nm) was prepared on the silicone monocrystalline substrate by vacuum vapor-deposition or sputtering, and stress was determined based on the radius of the curvature thereof.

2. Contact angle

An apparatus for measuring contact angles was used to determine the contact angle of water droplets.

3. Delamination

A film was formed on a polypropylene substrate in the same manner as in the case of the silicone substrate, the substrate was allowed to stand at room temperature for 24 hours, and the surface thereof was then observed under a microscope. The coefficient of linear expansion of the polypropylene substrate used was 12 x $10^{-5}$ (°$C^{-1}$),

The film stress, the contact angle, and delamination resulting from various materials and via various vapor-deposition techniques are shown in Table 1 and Fig. 2.

Table 1:

| Vapor-deposition techniques | Material | Film stress | Contact angle | Delamination |
|---|---|---|---|---|
| | | $\sigma \times 10^{21}$ (dyn/$cm^2$) | ° | |
| Sputtering | $SiO_2$ | 7.0 | 5 | Occurred |
| Vapor-deposition | $SnO_2 \cdot SiO_2$ | 5.4 | 5 | Cracked |
| | SiO | 3.1 | 10 | None |
| | $SiO_2$ | 3.8 | 5 | None |
| | $MgO \cdot SiO_2$ | 2.3 | 12 | None |
| | $2MgO \cdot SiO_2$ | 1.5 | - | None |
| | $MnO2 \cdot SiO_2$ | 1.2 | 13 | None |
| | $2MnO_2 \cdot SiO_2$ | 0.3 | 13 | None |
| | $NiO \cdot SiO_2$ | 3.8 | - | None |
| | $2NiO \cdot SiO_2$ | 0.1 | - | None |
| | $GeO_2 \cdot SiO_2$ | 1.8 | 12 | None |
| Sputtering | $MgO \cdot SiO_2$ | 3.1 | - | None |
| | $MgO \cdot SiO_2 \cdot Al_2O_3$ | 1.5 | 12 | None |

As is apparent from these results, the film stress of a film having hydrophilic properties and satisfactory adhesiveness is less than 5.4 x $10^{21}$ dyn/$cm^2$.

Example 1

This example relates to the capacity for carboxylic-acid-containing polymer binding and for protein immobilization on a plastic substrate.

(Formation of $SiO_2$ thin film on the surface of the plastic substrate)

SiO was vacuum vapor-deposited on the surface of melt-molded PMMA (VH, Mitsubishi Chemical Corporation) in the presence of oxygen to form a 100-nm $SiO_2$ film.

The thermal expansion coefficient ($K^{-1}$) of the PMMA substrate was 7 x $10^{-5}$. The film stress of the $SiO_2$ membrane was 3,8 x $10^{21}$ dyn/$cm^2$,

(Introduction of amino group)

3-Aminopropyltriethoxysilane (0.1 g) was dissolved in 100 ml of a solvent mixture comprising 0.01 N aqueous hydrochloric acid solution and ethanol (10:90, vol:vol). The resulting solution was kept at 50°C for 15 minutes, the above sample was soaked therein, and the reaction was allowed to proceed at 50°C for 12 minutes to introduce an amino group onto the $SiO_2$ surface.

(Preparation of Sample 1: introduction of carboxymethyldextran)

CMD (molecular weight: 1,000,000, Meito Sangyo Co., Ltd.) was dissolved in ultrapure water to 0.5% by weight thereof, a mixed solution of EDC (0.4 M)/NHS (0.1 M) was added thereto in an amount that was determined as the amount that would allow 2% of carboxyl groups to be activated when the total amount of the solution underwent reaction, and the mixture was then agitated at room temperature for 5 minutes. On the substrate upon which an amino group had been introduced, 200 μl of a CMD solution that had been actively esterified was added dropwise, and the substrate was spin-coated at 7,000 rpm for 45 seconds to form an actively esterified carboxymethyldextran thin film. Alter the reaction was allowed to proceed at room temperature for 1 hour, the substrate was cleaned once with 0.1 N NaOH and once with ultrapure water to obtain Sample 1 comprising a surface upon which carboxymethyldextran had been bound.

(Preparation of Sample 2)

Sample 2 was prepared in completely the same manner as in the case of Sample 1, except that polycarbonate (AD5503, Teijin) was used instead of PMMA.

The thermal expansion coefficient ($K^{-1}$) of the polycarbonate substrate was 6.5 x $10^{-5}$.

(Preparation of Sample 3)

Sample 3 was prepared in completely the same manner as in the case of Sample 1, except that Zeonex (330R, Nippon Zeon Co., Ltd.) was used instead of PMMA.

The thermal expansion coefficient ($K^{-1}$) of the Zeonex substrate was 7 x $10^{-5}$,

(Protein binding)

Areas each about 1 cm in diameter of Samples 1 to 3 were activated by EDC/NHS. Thereafter, the solution ofCy5-labeled CA (carbonic anhydrase, 50 μg/ml, pH 7.4) described in JP Patent Application No. 2005-277340 was brought into contact with an area about 1 cm in diameter, which was located at a distance of about 5 mm from the EDC/NHS-activated area for 5 minutes. This surface was cleaned twice with ultrapure water and twice with an aqueous solution of 10 mM NaOH, and the two-dimensional fluorescent images on the sample surface were analyzed at an excitation wavelength of 635 nm and a detection wavelength of 675 nm using FLA8000 (FUJI FILM Corporation),

As a result, fluorescent intensity was found to have been increased only at the EDC/NHS-activated site that had been in contact with a Cy5-labeled CA solution on the surfaces of Samples 1 to 3. This indicates that a carboxyl group of carboxymethyldextran bound to the substrate surface was actively esterified, an amide bond was formed with an amino group of a protein, and a protein was immobilized on the plastic substrate via carboxymethyldextran.

It was demonstrated that the present invention enabled the production of a surface capable of covalently binding a protein to a hydrophobic plastic.

Example 2

This example relates to the binding of a polyethylene glycol derivative to a plastic substrate and the capacity for inhibiting nonspecific adsorption thereof.

(Preparation of Sample 4)

Solution A:

Solution A: Ethanol (54.9 g), acetylacetone (2,46 g), and tetraethoxytitanium (2.82 g) were added to a 100-ml beaker, the mixture was agitated at room temperature for 10 minutes, 0.45 g of ultrapure water was added thereto, and the resultant was agitated at room temperature for an additional 60 minutes.

Solution B:

Ultrapure water (44.12 g) and Polymer 4 (n = 31, 1.73 g) were added to a 100-ml beaker, the mixture was agitated and dissolved, Solution A (10.10 g) and tetramethoxysilane (5.20 g) were added thereto, and the resultant was agitated.

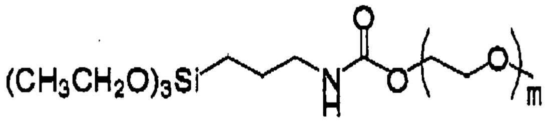

**Polymer 4**

Ultrapure water (1.0 g) was added to Solution B (3.23 g), the mixture was agitated, and the resultant was used as a coating liquid, The resulting coating liquid (300 μl) was added dropwise to a 100-nm $SiO_2$ film which was prepared by vacuum vapor-depositing SiO on the surface of the meld-molded PMMA (VH, Mitsubishi Chemical Corporation) in the presence of oxygen, the substrate was spin-coated at 300 rpm for 5 seconds and 7,000 rpm for 20 seconds, and the substrate was then heated at 100°C for 10 minutes to obtain Sample 4.

The thermal expansion coefficient ($K^{-1}$) of the PMMA substrate was 7 x $10^{-5}$. The film stress of the $SiO_2$ film was 3.8 x $10^{21}$ ($dyn/cm^2$),

(Preparation of Sample 5)

Sample 5 was prepared in completely the same manner as in the case of Sample 4, except that polycarbonate (AD5503, Teijin Corporation) was used instead of PMMA.

The thermal expansion coefficient ($K^{-1}$) of the polycarbonate substrate was 6.5 x $10^{-5}$.

(Preparation of Sample 6)

Sample 6 was prepared in completely the same manner as in the case of Sample 4, except that Zeonex (330R, Nippon Zeon Co., Ltd.) was used instead of PMMA.

The thermal expansion coefficient ($K^{-1}$) of the Zeonex substrate was 7 x $10^{-5}$,

(Evaluation of adsorptive properties)

An aqueous solution of a fluorescent protein, avidin-FITC (5 μl, 0.25 mg/ml, pH 5.0, acetate) or a solution of hydrophobic dye 1 in methanol (1 μl, concentration: 0.6%) was added dropwise to unmodified PMMA, polycarbonate, Zeonex, a glass slide, and Samples 4 to 6. The resultants were allowed to stand at room temperature for 5 minutes and then cleaned with ultrapure water. The cleaned sample surfaces were subjected to measurement of fluorescent intensity using FLA8000 (Fuji Photo Film Co., Ltd.) at a detection wavelength of 473 nm, using a filter 530DF20, at a resolution of 20 μm, and with a scan mode of 400 mm/s. The value obtained by subtracting the background fluorescent intensity was designated as an indicator for adsorption. The results are shown in Table 2.

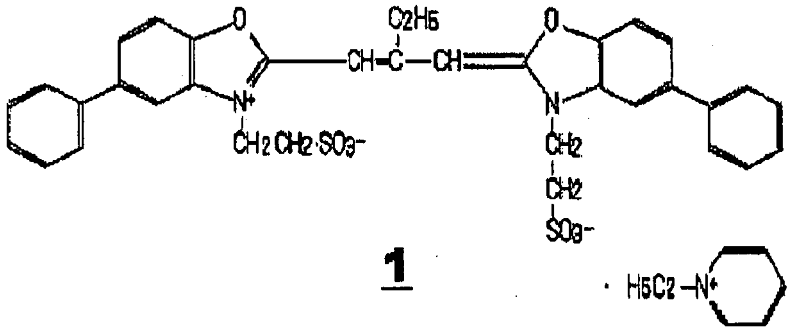

1

Table 2:

| | Avidin-FITC | Compound 1 | Remarks |
|---|---|---|---|
| PMMA | 751 | 1595792 | Comparative example |
| polycarbonate | 15425 | 1590507 | Comparative example |
| Zeonex | 9534 | 3043 | Comparative example |
| Glass slide | 8818 | 107 | Comparative example |
| Sample 4 | 29 | 449 | Present invention |
| Sample 5 | 0 | 490 | Present invention |
| Sample 6 | 78 | 100 | Present invention |

The amount of the avidin-FITC protein adsorbed to PMMA was small; however, the amount of the hydrophobic compound 1 adsorbed thereto was very large. The amount of avidin-FITC and that of the hydrophobic compound 1 adsorbed to polycarbonate were both very large. Although the amount of the hydrophobic compound 1 adsorbed to cycloolefin polymer (Zeonex) was small, that of avidin-FITC adsorbed thereto was very large. The amount of the hydrophobic compound 1 adsorbed to glass was small; however, that of avidin-FITC adsorbed thereto was very large. Thus, all samples were found to be insufficient as adsorption-inhibiting surfaces because of the large amount of a protein, hydrophobic low-molecular weight compound, or both thereof adsorbed thereto, In contrast, Samples 4 to 6 to which polyethylene glycol derivatives had been bound were found to be capable of effectively inhibiting adsorption of both of a protein and a hydrophobic low-molecular weight compound.

According to the present invention, it was demonstrated that a surface capable of effectively inhibiting adsorption of a protein and a hydrophobic low-molecular weight compound could be formed on a hydrophobic plastic.

Effect of the Invention

The present invention enables the provision of a biochemical instrument wherein an adhesive layer that has hydrophilic properties, heat resistance, and satisfactory adhesive properties is provided between the plastic material surface and the hydrophilic polymer layer. Further, the present invention enables the provision of a biochemical instrument having surfaces that can inhibit adsorption of biopolymers and of hydrophobic low-molecular-weight compounds.

## Claims

1. A biochemical instrument comprising a plastic material that is coated on its surface with a hydrophilic polymer, which comprises a metal oxide thin film between the plastic material and the hydrophilic polymer layer, wherein the film stress of said metal oxide thin film is less than $5.4 \times 10^{21}$ dyn/cm$^2$.

2. The biochemical instrument of claim 1 wherein the plastic material has a thermal expansion coefficient ($K^{-1}$) of 1.3 to $15 \times 10^{-5}$.

3. The biochemical instrument of claim 1 wherein the plastic material is made from polymethylmethacrylate (PMMA), polycarbonate (PC), cycloolefin polymer (COP), polyethylene naphthalate (PEN), polyethylene terephthalate (PET), polypropylene (PP), polyimide (PI), liquid crystal polyester (LCP), or polydimethylsiloxane (PDMS).

4. The biochemical instrument of claim 1 wherein the metal oxide is $SiO_2$, $Al_2O_3$, $TiO_2$, $MnO_2$, MgO, $SnO_2$, NiO, $GeO_2$, or a mixture thereof.

5. The biochemical instrument of claim 1 wherein the thickness of the metal oxide film is between 10 nm and 500 nm.

6. The biochemical instrument of claim 1 wherein the hydrophilic polymer layer has a portion that inhibits adsorption of hydrophobic low-molecular-weight compounds and a portion that inhibits adsorption of biopolymers.

7. The biochemical instrument of claim 6 wherein the portion that inhibits adsorption of hydrophobic low-molecular-weight compounds is composed of an inorganic crosslinking polymer, an organic crosslinking polymer, or an organic/inorganic hybrid polymer.

8. The biochemical instrument of claim 6 wherein the portion that inhibits adsorption of hydrophobic low-molecular-weight compounds comprises a hydrolytic condensate of metal alkoxide.

9. The biochemical instrument of claim 6 wherein the portion that inhibits adsorption of biopolymers is composed of a hydrophilic polymer,

10. The biochemical instrument of claim 9 wherein the hydrophilic polymer is a non-charged hydrophilic polymer.

11. The biochemical instrument of claim 10 wherein the non-charged hydrophilic polymer is any of polyethylene glycol, polyvinylpyrrolidone, polyvinyl alcohol, poly(hydroxylalkyl)methacrylate, polyacrylamide, MPC polymer, dextran, agarose, pullulan, or polypeptide.

12. The biochemical instrument of claim 6 wherein the portion that inhibits adsorption of hydrophobic low-molecular-weight compounds and the portion that inhibits adsorption of biopolymers each have a structure represented by the following formula:

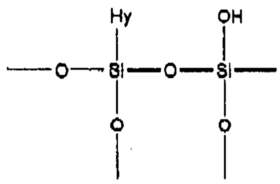

wherein Hy represents a hydrophilic polymer.

13. The biochemical instrument of claim 12 wherein Hy is any of polyethylene glycol, polyvinylpyrrolidone, polyvinyl alcohol, poly(hydroxylalkyl)methacrylate, polyacrylamide, MPC polymer, dextran, agarose, pullulan, or polypeptide.

14. The biochemical instrument of claim 1 which is obtained by coating the surface of the plastic material with a solution containing a polymer represented by formula (I) or (II) and allowing the polymer to crosslink,

Formula (I)

$$(R^1)_x(OR^2)_{3-x}Si-L_1-(A)-L_2-Si(OR^3)_{3-y}(R^4)_y$$

Formula (II)

$$(R^5)_z(OR^6)_{3-z}Si-L_3-(B)-R^7$$

wherein $R^1$ to $R^6$ each independently represent a hydrogen atom or a hydrocarbon group containing 8 or fewer carbon atoms; $R^7$ represents a hydrogen atom or a monovalent nonmetal atomic group; x, y, and z each independently are an integer from 0 to 2; $L^1$ to $L^3$ each independently represent a divalent linking group containing 3 or more types of atoms selected from among carbon, hydrogen, oxygen, nitrogen, and sulfur atoms; and A and B each independently represent a polymer or an oligomer having structural unit repeats.

**15.** A biosensor which comprises the biochemical instrument of claim 1.

Fig. 1

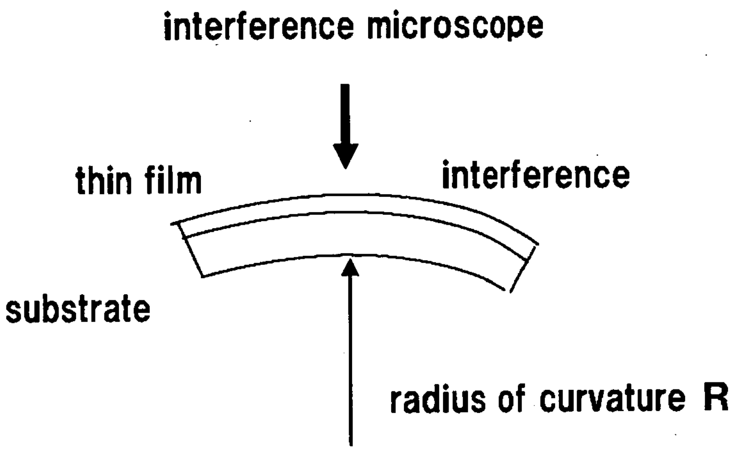
interference microscope
thin film
interference
substrate
radius of curvature R

Fig.2

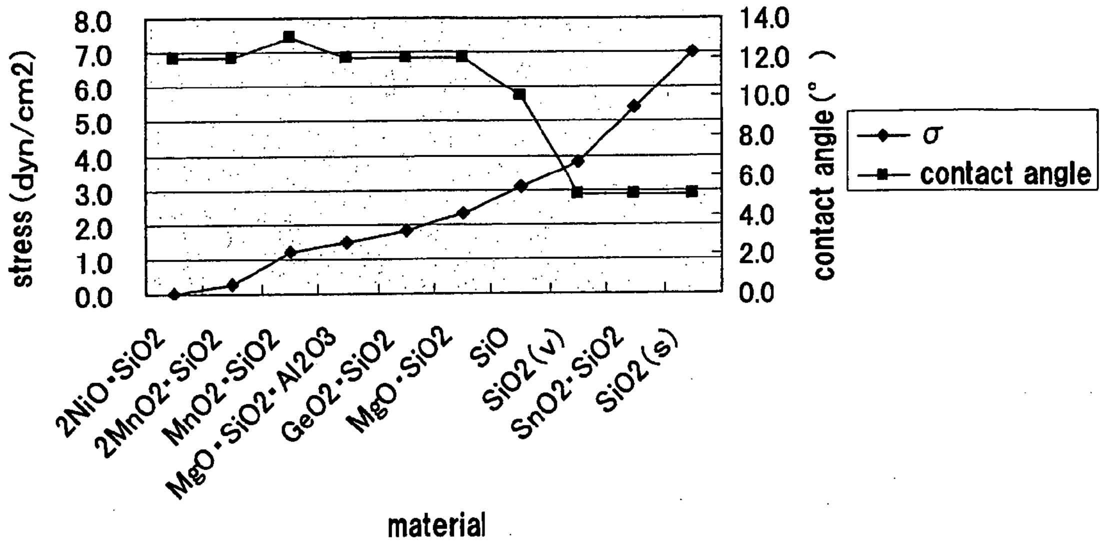
8.0
7.0
6.0
5.0
4.0
3.0
2.0
1.0
0.0
stress (dyn/cm2)
14.0
12.0
10.0
8.0
6.0
4.0
2.0
0.0
contact angle (° )
σ
contact angle
2NiO·SiO2
2MnO2·SiO2
MnO2·SiO2
MgO·SiO2·Al2O3
GeO2·SiO2
MgO·SiO2
SiO
SiO2(v)
SnO2·SiO2
SiO2(s)
material

European Patent Office

# EUROPEAN SEARCH REPORT

Application Number
EP 07 01 8939

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 05 132574 A (SEKISUI CHEMICAL CO LTD) 28 May 1993 (1993-05-28) and English computer translation provided for by the Japanese Patent Office http://dossier1.ipdl.inpit.go.jp/AIPN/aipn_call_transl.ipdl?N0000=7413&N0120=01&N2001=2&N3001=H05-132574 * abstract * | 1-14 | INV. C08J7/04 G01N33/53 A61J1/00 A61M3/00 |
| Y | | 1-14 | |
| Y | EP 0 989 418 A (JOHNSON & JOHNSON VISION PROD [US]) 29 March 2000 (2000-03-29) * claims 1-10 * | 1-14 | |
| Y | JACOB PIEHLER ET AL: "Surface modification for direct immunoprobes" BIOSENSORS & BIOELECTRONICS, vol. 11, no. 6/7, 1996, pages 579-590, XP002466758 * page 779 - page 584 * | 1-14 | |
| A | JP 06 316025 A (SHOWA DENKO KK; SHOWA HIGHPOLYMER) 15 November 1994 (1994-11-15) and English computer translation provided for by the Japanese Patent Office http://dossier1.ipdl.inpit.go.jp/AIPN/aipn_call_transl.ipdl?N0000=7413&N0120=01&N2001=2&N3001=H06-316025 * abstract * | 1-14 | TECHNICAL FIELDS SEARCHED (IPC): C08J G01N A61J A61M G02B |
| X | US 2006/210425 A1 (MIRKARIMI LAURA [US]) 21 September 2006 (2006-09-21) * paragraphs [0001], [0004] - [0006], [0074]; claims 1-31 * | 1-14 | |

The present search report has been drawn up for all claims

| Place of search | Date of completion of the search | Examiner |
|---|---|---|
| Munich | 30 January 2008 | olde Scheper, Bernd |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

1 EPO FORM 1503 03.82 (P04C01)

**ANNEX TO THE EUROPEAN SEARCH REPORT ON EUROPEAN PATENT APPLICATION NO.** EP 07 01 8939

This annex lists the patent family members relating to the patent documents cited in the above-mentioned European search report.
The members are as contained in the European Patent Office EDP file on
The European Patent Office is in no way liable for these particulars which are merely given for the purpose of information.

30-01-2008

| Patent document cited in search report | Publication date | Patent family member(s) | Publication date |
|---|---|---|---|
| JP 5132574 A | 28-05-1993 | NONE | |
| EP 0989418 A | 29-03-2000 | AU 757262 B2 | 13-02-2003 |
| | | AU 4876799 A | 30-03-2000 |
| | | BR 9904255 A | 17-04-2001 |
| | | CA 2283041 A1 | 23-03-2000 |
| | | CN 1249443 A | 05-04-2000 |
| | | JP 2000137195 A | 16-05-2000 |
| | | KR 20000034933 A | 26-06-2000 |
| | | SG 85676 A1 | 15-01-2002 |
| | | TW 229759 B | 21-03-2005 |
| | | US 6099852 A | 08-08-2000 |
| JP 6316025 A | 15-11-1994 | JP 3012756 B2 | 28-02-2000 |
| US 2006210425 A1 | 21-09-2006 | WO 2006101995 A2 | 28-09-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5132574 A
- JP 2000137195 A
- JP 2004314073 A
- JP 2000301837 A
- JP 5230101 A
- JP 8120003 A
- JP 2004058566 A
- JP 9087395 A
- JP 2005277340 A


### Non-patent literature cited in the description


- Saibou-seibutsugaku-jiten (Encyclopedia of cell biology. Asakura Publishing Co., Ltd, 148-149